(19) **Europäisches Patentamt**

**European Patent Office**

**Office européen des brevets**

(11) **EP 0 532 432 B1**

(12) **FASCICULE DE BREVET EUROPEEN**

(45) Date de publication et mention
de la délivrance du brevet:
**06.03.1996 Bulletin 1996/10**

(51) Int. Cl.$^6$: **A61M 1/16**, A61M 1/34

(21) Numéro de dépôt: **92420299.7**

(22) Date de dépôt: **09.09.1992**

(54) **Rein artificiel**

Kunstniere

Artificial kidney

(84) Etats contractants désignés:
**AT BE CH DE ES FR GB IT LI NL SE**

(30) Priorité: **10.09.1991 FR 9111351**

(43) Date de publication de la demande:
**17.03.1993 Bulletin 1993/11**

(60) Demande divisionnaire: **95202060.0**

(73) Titulaire: **HOSPAL INDUSTRIE**
**F-69883 Meyzieu Cédex (FR)**

(72) Inventeurs:
- **Chevallet, Jacques**
  **F-69360 Serezin du Rhone (FR)**
- **Béné, Bernard**
  **F-69540 Irigny (FR)**

(56) Documents cités:
EP-A- 0 192 588          EP-A- 0 256 956
EP-A- 0 321 754          DE-A- 2 703 188
FR-A- 2 332 031

- ASAIO TRANSACTIONS. vol. 32, no. 1, 1 Juillet
  1986, HAGERSTOWN, MD US pages 422 - 424
  M.FERIANI 'buffer balance in bicarbonate
  hemodiafiltration'

## Description

La présente invention a pour objet un rein artificiel qui permette le dosage de substances dans le sang et qui soit en outre particulièrement adapté au traitement de personnes ayant perdu temporairement l'usage de leurs reins à la suite d'un accident ou d'une opération chirurgicale.

Ces substances peuvent être, par exemple, des médicaments (antibiotiques, notamment), du glucose, ou certains électrolytes du sang (potassium, magnésium, bicarbonate, en particulier). Dans la suite, on décrira l'invention dans son application au dosage du bicarbonate, mais il va de soi que cet exemple d'utilisation, donné à titre purement illustratif, ne saurait être considéré comme limitatif.

On sait que les reins, outre des fonctions d'épuration des déchets plasmatiques (urée, créatinine) et d'excrétion hydrique, jouent un rôle important dans le maintien de l'équilibre acido-basique du milieu interne, notamment en éliminant des acides faibles (phosphates, acides monosodiques) et en produisant des sels d'ammonium.

Chez les personnes qui ont perdu l'usage de leurs reins, temporairement ou définitivement, ce mécanisme régulateur ne jouant plus, on constate une élévation de l'acidité du milieu intérieur (acidose), c'est-à-dire aussi une baisse du pH du sérum sanguin, qui se déplace vers 7 (le pH sanguin varie normalement dans les limites très étroites qui vont de 7,35 à 7,45).

De façon classique, pour pallier la déficience du mécanisme régulateur rénal, on agit sur un autre mécanisme régulateur de l'équilibre acido-basique du milieu interne, qui est constitué par les systèmes tampons du sang, dont le principal comprend l'acide carbonique, comme acide faible, associé à son sel alcalin, le bicarbonate. C'est ainsi que, pour combattre l'acidose d'une personne souffrant d'insuffisance rénale, on fait passer du bicarbonate dans son sang, généralement simultanément à une séance d'épuration du sang par hémodiafiltration ou par hémodialyse.

Lors d'un traitement par hémofiltration, où le sang est épuré par ultrafiltration d'eau plasmatique au travers d'une membrane semi-perméable avec transfert convectif simultané de déchets plasmatiques, l'apport en bicarbonate se fait par perfusion d'une solution de bicarbonate de sodium.

Lors d'un traitement par hémodialyse, où le sang est épuré par transfert diffusif de déchets plasmatiques au travers d'une membrane semi-perméable dont la face non baignée par le sang est irriguée par un flux de liquide de dialyse, l'apport en bicarbonate peut se faire de deux façons, selon que le liquide de dialyse contient du bicarbonate ou qu'il en est dépourvu.

Dans le premier cas, l'apport au sang de bicarbonate se fait par diffusion au travers de la membrane semi-perméable, du liquide de dialyse vers le sang, la concentration en bicarbonate dans le liquide de dialyse étant ajustée en conséquence.

Dans le second cas, comme lors du traitement par hémofiltration, une solution de bicarbonate de sodium est perfusée au patient en quantité suffisante pour compenser les pertes diffusives (ou convectives, en hémofiltration) qui se produisent dans l'échangeur à membrane ainsi que le déficit dont souffre le malade en état d'acidose.

Le document EP 0 192 588 décrit un procédé de dialyse où le liquide de traitement qui est mis en circulation dans le dialyseur contient du bicarbonate de sodium et, éventuellement, d'autres électrolytes du sang à l'exception de ceux qui précipitent avec le bicarbonate, et où un liquide dépourvu de bicarbonate et contenant les électrolytes du sang qui précipitent avec le bicarbonate est simultanément perfusé au patient.

La concentration finale en bicarbonate du sang d'un patient soumis à l'un de ces traitements dépend de la concentration en bicarbonate de la solution de perfusion ou du liquide de dialyse, de leurs débits respectifs, et du débit du sang du patient circulant dans un circuit pour circulation extracorporelle de sang comprenant l'échangeur à membrane. Actuellement, à l'exception de la concentration de la solution de bicarbonate de sodium, qui est fixée par le fabricant, ces paramètres sont déterminés empiriquement par le médecin, à partir de mesures du pH sanguin faites régulièrement dans le cas des patients en état de choc, dont le sang est dialysé ou ultrafiltré en permanence, ou faites après une séance de traitement et avant la séance suivante, dans le cas des patients ayant définitivement perdu l'usage de leurs reins. Il en résulte que la concentration en bicarbonate du sang du patient correspond rarement exactement à la concentration souhaitée.

Un but de l'invention est de réaliser un rein artificiel qui permette d'agir avec précision sur l'équilibre acido-basique d'un patient souffrant d'insuffisance rénale.

Selon l'invention, pour atteindre ce but, on prévoit un rein artificiel comprenant :

- un échangeur ayant deux compartiments séparés par une membrane semi-perméable, un premier compartiment étant relié à un circuit pour circulation extracorporelle de sang connectable à un patient, un second compartiment ayant une sortie pour l'évacuation d'un liquide usé,
- des moyens pour perfuser au patient un liquide de perfusion contenant une substance (A),

caractérisé en ce qu'il comprend des moyens de dosage (22, 25) pour ajuster la concentration de la substance (A) dans le sang du patient (9) à une concentration souhaitée $[A]_{DES}$, compte tenu du transfert diffusif et/ou convectif de la substance (A) au travers de la membrane (4) de l'échangeur (1), ces moyens de dosage comprenant des moyens (22, 25) pour régler le débit du liquide de perfusion ($Q_A$) en fonction du débit ($Q_{OUT}$) de liquide usé.

Selon une caractéristique de l'invention, le débit $(Q_A)$ de perfusion de la solution stérile et le débit $(Q_{OUT})$ du liquide usé sont liés par la formule :

$$Q_A = \frac{[A]_{DES}}{[A]_{SOL}} \times Q_{OUT}$$

ou par la formule :

$$Q_A = \frac{[A]_{DES}}{[A]_{SOL}} \times Cl$$

dans lesquelles $[A]_{SOL}$ est la concentration de la substance (A) dans la solution stérile et Cl est la clairance du rein artificiel pour la substance (A).

Selon une autre caractéristique de l'invention, la substance (A) étant du bicarbonate, le rein artificiel comprend une source de liquide de dialyse/substitution ne contenant pas de bicarbonate reliée au circuit pour circulation extracorporelle de sang et à une entrée du second compartiment de l'échangeur, et des moyens d'occlusion pour ou bien isoler la source, ou bien permettre l'écoulement du liquide de dialyse/substitution dans le circuit pour circulation extracorporelle de sang, ou bien permettre l'écoulement du liquide de dialyse/substitution dans le second compartiment de l'échangeur.

Selon encore une autre caractéristique de l'invention, le rein artificiel comprend des moyens pour faire un bilan du (ou des) liquides(s) introduit(s) dans le circuit pour circulation extracorporelle de sang et du liquide usé (ultrafiltrat et/ou liquide de dialyse usé) s'écoulant du second compartiment de l'échangeur. Ces moyens pour faire le bilan des liquides peuvent comprendre une balance pour peser un réservoir constituant une source de liquide de dialyse/substitution et un réservoir pour le recueil du liquide usé, et une balance pour peser un réservoir constituant une source de liquide contenant la substance (A).

D'autres caractéristiques et avantages de l'invention apparaîtront à la lecture de la description qui va suivre. On se reportera aux dessins annexés sur lesquels :

la figure 1 est un schéma simplifié d'un premier mode de réalisation de l'invention.
la figure 2 est un schéma simplifié d'un second mode de réalisation de l'invention.

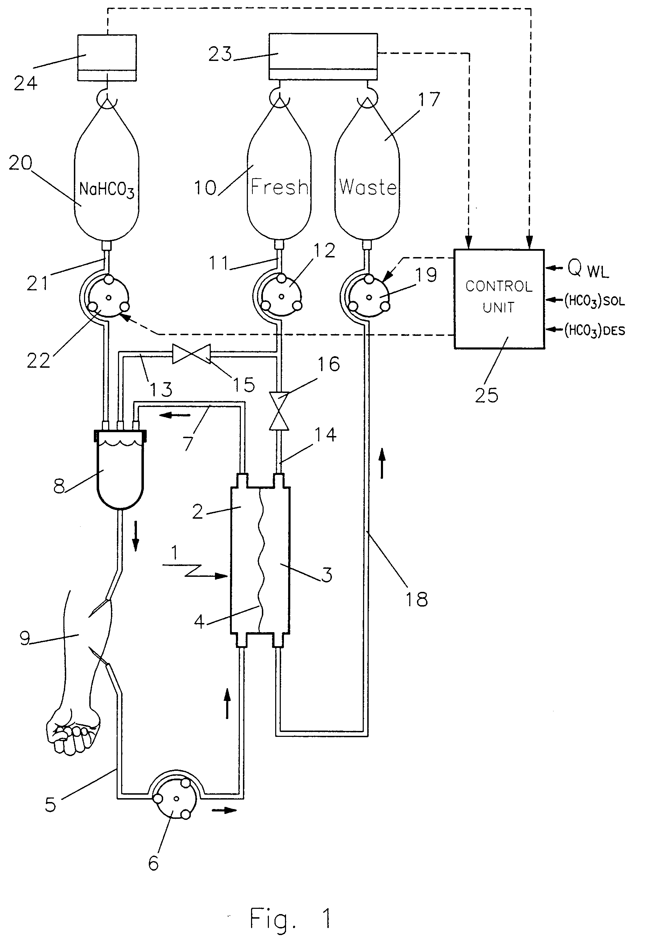

Le rein artificiel représenté sur la figure 1 comprend un échangeur 1 ayant deux compartiments 2, 3 séparés par une membrane semi-perméable 4. Le compartiment 2 est connecté à un circuit pour circulation extracorporelle de sang comprenant une canalisation amont 5, sur laquelle est disposée une pompe de circulation 6, et une canalisation aval 7 équipée d'un piège à bulles 8. Les canalisations 5 et 7 sont respectivement munies à leur extrémité libre d'une aiguille ou d'un connecteur pour cathéter, pour le raccordement du circuit pour circulation extracorporelle de sang au système vasculaire d'un patient 9.

Un réservoir 10 de liquide de dialyse/substitution stérile et dépourvu de bicarbonate est relié, par l'intermédiaire d'une portion de canalisation commune 11 sur laquelle est disposée une pompe de circulation 12, à deux canalisations 13, 14 connectées respectivement au piège à bulles 8 et à une entrée du second compartiment 3 de l'échangeur 1. Des moyens d'occlusion 15, 16, tels que des clamps électromagnétiques, sont prévus respectivement sur les canalisations 13, 14 pour permettre de relier sélectivement le réservoir 10 à l'échangeur 1 ou au circuit pour circulation extracorporelle de sang.

Un second réservoir 17 pour liquide usé (ultrafiltrat et/ou liquide de dialyse usé) est relié à une sortie du second compartiment 3 de l'échangeur 1, par une canalisation 18 sur laquelle est disposée une pompe d'extraction 19 de liquide usé. La pompe 19 permet de créer une dépression variable dans le compartiment 3 de l'échangeur 1, c'est-à-dire aussi de faire varier la pression transmembranaire et, par conséquent, le débit d'ultrafiltration.

Un troisième réservoir 20 de solution stérile de bicarbonate de sodium est relié au piège à bulles 8, par l'intermédiaire d'une canalisation 21 sur laquelle est disposée une pompe de circulation 22.

Conformément à l'invention, le rein artificiel représenté sur la figure 1 comprend des moyens pour établir un bilan du (des) liquide(s) perfusé(s) au patient 9 et des liquides usés, pour programmer éventuellement une perte de poids souhaitée par extraction d'eau plasmatique en excès par rapport au(x) liquide(s) perfusé(s), et pour ajuster la concentration en bicarbonate du plasma du patient à une valeur déterminée. Ces moyens comprennent une première balance 23 pour peser le réservoir 10 de liquide de dialyse/substitution et le réservoir 17 de liquide usé, une seconde balance 24 pour peser le réservoir 20 de solution de bicarbonate de sodium, et une unité de commande 25 pouvant recevoir, comme signaux d'entrée, les informations fournies par les balances 23, 24, une consigne $Q_{WL}$ de débit de perte de poids souhaité, la valeur $[HCO_3]_{SOL}$ de la concentration en bicarbonate de la solution contenue dans le réservoir 20 et une consigne $[HCO_3]_{DES}$ de concentration du sang en bicarbonate souhaitée. L'unité de commande 25 est prévue pour piloter la pompe 19 d'extraction de liquide usé, compte tenu du débit de perte de poids désiré $Q_{WL}$ et du débit $Q_{IN}$ imposé à la pompe 12 de circulation du liquide de dialyse/substitution, et pour piloter la pompe 22 de perfusion de la solution de bicarbonate compte tenu du débit $Q_{OUT}$ de la pompe 19 d'extraction de liquide usé.

Conformément à l'invention, l'asservissement du débit $Q_{HCO3}$ de la pompe de perfusion 22 au débit $Q_{OUT}$ de la pompe d'extraction 19 peut être défini, quel que soit le type de traitement auquel est soumis le patient, hémofiltration avec ou sans perfusion de liquide de substitution, hémodialyse ou hémodiafiltration, par la formule :

$$Q_{HCO3} = Q_{OUT} \times \frac{[HCO_3]_{DES}}{[HCO_3]_{SOL}} \qquad (1)$$

Le fonctionnement du rein artificiel qui vient d'être décrit est le suivant :

En mode hémofiltration sans perfusion de liquide de substitution, les clamps 15, 16 sont fermés, la pompe 12 de circulation du liquide de dialyse/substitution est à l'arrêt et les pompes 19, 22 d'extraction de filtrat sanguin et de perfusion de solution de bicarbonate tournent. L'unité de commande 25 règle en continu le débit $Q_{OUT}$ de la pompe d'extraction 19, mesuré au moyen de la balance 23, de façon que ce débit soit égal en permanence à la somme du débit $Q_{WL}$ de perte de poids souhaité et du débit $Q_{HCO3}$ de perfusion de la solution de bicarbonate, mesuré au moyen de la balance 24. L'unité de commande 25 règle aussi en continu le débit $Q_{HCO3}$ de la pompe 22 de solution de bicarbonate, en fonction de la concentration en bicarbonate $[HCO_3]_{DES}$ souhaitée du sang du patient, de la concentration $[HCO_3]_{SOL}$ de la solution contenue dans le réservoir 20, et de la perte convective se produisant dans l'échangeur 1, qui est égale à $Q_{OUT} \times [HCO_3]_{BLD}$, $[HCO_3]_{BLD}$ étant la concentration en bicarbonate du sang du patient et la transmittance des membranes haute perméabilité utilisées en hémofiltration étant égale à 1 pour les électrolytes du sang (on rappelle que la formule générale donnant le débit massique Js d'une substance traversant une membrane en fonction du débit volumétrique Jv d'eau plasmatique est la suivante :

$$Js = Jv \times Tr \times Cs$$

où Cs est la concentration de la substance dans le sang et où Tr est la transmittance de la membrane vis à vis de cette substance.)

L'asservissement de la pompe 22 de perfusion de bicarbonate selon la formule (1) donnée plus haut permet donc d'amener progressivement le sang du patient 9 à un état d'équilibre où la concentration en bicarbonate est égale à $[HCO_3]_{DES}$.

En mode hémofiltration avec perfusion de liquide de substitution, le clamp 16 est fermé, le clamp 15 est ouvert et les trois pompes 12, 19, 22 tournent, le débit de la pompe 12 étant fixé par l'opérateur à une valeur constante en début de séance de traitement. Le fonctionnement du rein artificiel pour ce second mode de traitement ne diffère pas de celui qui vient d'être décrit plus haut, à ceci près que, pour piloter la pompe d'extraction 19, l'unité de commande 25 prend en compte la vidange du réservoir 10, le débit $Q_{OUT}$ imposé à la pompe 19 étant alors choisi pour que la différence entre le débit de liquide de substitution et le débit de liquide usé, mesurée par la balance 23, soit égale à la somme du débit $Q_{WL}$ de perte de poids souhaité et du débit $Q_{HCO3}$ de perfusion de solution de bicarbonate, mesuré par la balance 24. Le réglage de la pompe 22 de perfusion de solution de bicarbonate est effectué comme précédemment selon l'asservissement décrit par la formule (1).

En mode hémodialyse, le clamp 15 est fermé, le clamp 16 est ouvert et les trois pompes 12, 19, 22 tournent. L'unité de commande 25 règle en continu le débit $Q_{OUT}$ de la pompe d'extraction 19, de façon que la différence entre le débit de liquide de dialyse et le débit de liquide usé, mesurée par la balance 23, soit égale en permanence au débit $Q_{HCO3}$ de perfusion de solution de bicarbonate, mesuré par la balance 24, la consigne de débit de perte de poids étant nulle.

L'unité de commande 25 règle en outre le débit $Q_{HCO3}$ de perfusion de la solution de bicarbonate, en fonction de la concentration en bicarbonate $[HCO_3]_{DES}$ souhaitée du sang du patient, de la concentration $[HCO_3]_{SOL}$ de la solution contenue dans le réservoir 20, et de la perte diffusive dans l'échangeur 1, qui est égale à Cl $\times [HCO_3]_{BLD}$, $[HCO_3]_{BLD}$ étant la concentration en bicarbonate du sang du patient et Cl, la clairance du rein artificiel pour le bicarbonate (la clairance est définie, de façon générale, comme le rapport entre la quantité de substance éliminée par unité de temps et la concentration de la substance dans le sang à l'entrée de l'échangeur). Pour obtenir que la concentration en bicarbonate du sang atteigne, à l'équilibre, une valeur donnée $[HCO_3]_{DES}$, il faut donc asservir le débit $Q_{HCO3}$ de perfusion de la pompe 22 de solution de bicarbonate selon la formule :

$$Q_{HCO3} = Cl \times \frac{[HCO_3]_{DES}}{[HCO_3]_{SOL}} \qquad (2)$$

ce qui suppose une détermination préalable de la clairance du rein artificiel, laquelle dépend du type d'échangeur utilisé (nature de la membrane, surface) ainsi que, en général, des débits du sang et du liquide de dialyse dans l'échangeur.

Cependant, pour certaines valeurs de débits de sang et de liquide de dialyse, la clairance du rein pour une substance et un type d'échangeur donnés est sensiblement constante. C'est le cas en particulier lorsque, d'une part, la surface de la membrane de l'échangeur est suffisamment grande par rapport au débit du sang, et que, d'autre part, le débit du sang est relativement important par rapport au débit du liquide de dialyse (rapport de l'ordre de trois, et plus). Le sang et le liquide de dialyse ont alors, au sortir de l'échangeur, la même concentration en la substance considérée, et la clairance Cl est égale au débit de sortie du liquide usé, $Q_{OUT}$. En d'autres termes, dans ces conditions de fonctionnement particulières, l'asservissement de la pompe 22 de perfusion de solution de bicarbonate est défini par la formule (1). Or, ces conditions sont applicables au traitement par dialyse continue des patients en état de choc dont l'épuration doit se faire à un taux modéré pour pouvoir être supportée par des organismes affaiblis.

Le rein artificiel selon l'invention présente donc un intérêt particulier pour le traitement des patients ayant perdu l'usage temporaire de leurs reins, puisque, quel que soit le type de traitement auxquels ils sont soumis, ce rein artificiel permet, par la commande d'une seule pompe selon une formule d'asservissement unique, d'agir simplement sur leur équilibre acido-basique.

Ce rein artificiel peut aussi fonctionner en mode hémodiafiltration, dans lequel la position des clamps et le fonctionnement des pompes sont les mêmes que ceux qui sont adoptés en mode hémodialyse, à ceci près que la pompe 19 est pilotée pour provoquer une ultrafiltration dans le rein conformément à une consigne de débit de perte de poids donnée.

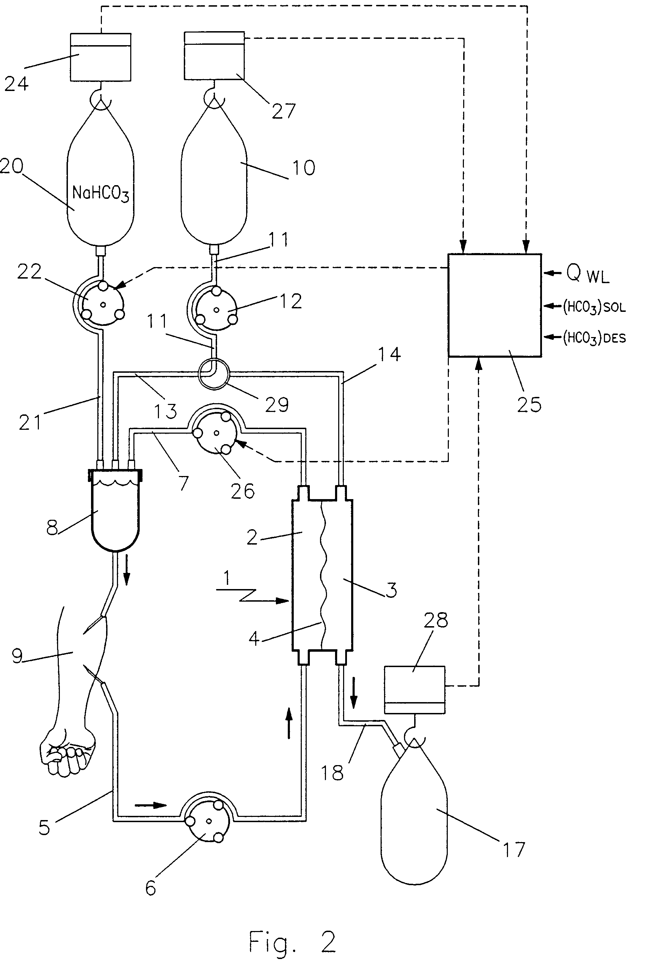

Le rein artificiel représenté sur la figure 2 se différencie de celui qui vient d'être décrit en ce que son circuit pour circulation extracorporelle de sang comprend une seconde pompe 26 disposée en aval de l'échangeur 1, pour permettre de faire varier la pression transmembranaire dans l'échangeur 1 et, par suite, le débit d'eau plasmatique ultrafiltrée (c'est-à-dire $Q_{OUT}$ en hémofiltration). Par ailleurs, les réservoirs 10, 17 de liquide de dialyse/substitution et de liquide usé sont pesés par des balances indépendantes 27, 28 et la canalisation 18 reliant le compartiment 3 de l'échangeur 1 au réservoir de liquide usé 17 est dépourvue de pompe.

En outre, une vanne trois voies 29, à laquelle sont raccordées les canalisations 11, 13 et 14, permet soit de relier le réservoir 10 de liquide de dialyse/substitution au piège à bulles 8, soit de relier le réservoir 10 au compartiment 3 de l'échangeur 1, soit d'isoler le réservoir 10.

Le fonctionnement de ce second mode de réalisation de rein artificiel selon l'invention ne diffère pas sensiblement de celui du précédent. En mode hémofiltration sans perfusion de liquide de substitution, la pompe 12 ne tourne pas et le débit de la pompe 26 est réglé par l'unité de commande 25 pour que le débit de filtration $Q_{OUT}$, mesuré par la balance 28, soit égal à la somme du débit de consigne de perte de poids $Q_{WL}$ et du débit de perfusion de la solution de bicarbonate $Q_{HCO3}$ mesuré par la balance 24.

En mode hémofiltration avec perfusion de liquide de substitution, la pompe 12 tourne à un débit réglé initialement par l'opérateur et le débit de la pompe 26 est asservi par l'unité de commande 25 pour que le débit de filtration $Q_{OUT}$ soit égal à la somme du débit de consigne de perte de poids $Q_{WL}$, du débit de perfusion de la solution de bicarbonate $Q_{HCO3}$ et du débit $Q_{IN}$ de perfusion du liquide de substitution, mesuré par la balance 27.

En mode de dialyse, les pompes 6 et 26 disposées sur le circuit sang, respectivement en amont et en aval de l'échangeur 1, tournent au même débit et la pompe 12, qui sert alors de pompe de circulation de liquide de dialyse, tourne à un débit réglé initialement par l'opérateur.

En mode hémodialfiltration, l'unité de commande 25 règle le débit de la pompe 26 comme en mode hémofiltration avec perfusion de liquide de substitution.

Sauf en mode hémofiltration, où elle ne tourne pas, le débit de la pompe 22 de circulation de liquide de dialyse/substitution est régulé par l'unité de commande 25 qui compare le débit souhaité mis initialement en mémoire dans cette unité et le débit mesuré au moyen de la balance 27. Quant à la pompe 22 de perfusion de bicarbonate, son débit $Q_{HCO3}$ est, comme précédemment, asservi au débit de liquide usé $Q_{OUT}$, mesuré par la balance 28, conformément à la formule (1) et, le cas échéant, à la formule (2).

L'invention n'est pas limitée aux modes de réalisation qui viennent d'être décrits et elle est susceptible de variantes.

En particulier, à la différence de ce qui est prévu dans les exemples de rein artificiel décrits plus haut, il est possible de prévoir que, dans les modes où du liquide de dialyse/substitution est mis en circulation par la pompe 12, ce soit le débit de la pompe 19 (26) commandant le débit d'ultrafiltration qui soit fixé initialement par l'opérateur et le débit de la pompe 12 qui soit asservi en fonction du bilan des liquides frais et usés, perfusés et ultrafiltrés et du débit de perte de poids souhaité.

Par ailleurs, la détermination du débit des liquides nécessaire à l'asservissement de la pompe 19 (26) réglant le débit d'ultrafiltration et à l'asservissement de la pompe 22 de perfusion de solution de bicarbonate, pourrait se faire avec d'autres moyens de mesure que des balances, par exemple avec des débitmètres ou des moyens de mesure volumétrique.

En outre, les pompes 12, 22 utilisées pour régler le débit des liquides de dialyse/substitution et de solution de bicarbonate pourraient être remplacées par des clamps électromagnétiques, les liquides s'écoulant par gravité.

Il va de soi aussi que la source 20 de liquide de perfusion pourrait être connectée directement au système vasculaire du patient 9 et non pas au circuit 5, 7 pour circulation extracorporelle de sang.

Enfin, comme cela a été mentionné plus haut, les moyens de dosage dont est équipé le rein artificiel selon l'invention peuvent être utilisés pour doser toutes sortes de substances dans le sang d'un patient soumis à une séance de traitement par hémofiltration, hémodialyse ou hémodialfiltration. Dans le cas d'un médicament A, par exemple, le réservoir 20 contient alors un soluté stérile de ce médicament, tandis que le réservoir 10 contient un liquide de dialyse dans lequel sont présents les principaux électrolytes du sang, bicarbonate compris. Le fonctionnement du rein n'est pas différent de celui qui a été décrit à propos des exemples de réalisation des figures 1 et 2, et, en particulier, l'asservissement de la pompe de perfusion 22 au débit de liquide usé se fait selon la formule (1), ou, le cas échéant, selon la formule (2).

**Revendications**

1. Rein artificiel comprenant :

   - un échangeur (1) ayant deux compartiments (2, 3) séparés par une membrane semi-perméable (4), un premier compartiment (2) étant relié à un circuit (5, 7) pour circulation extracorporelle de sang connectable à un patient (9), un second compartiment (3) ayant une sortie pour l'évacuation d'un liquide usé,

- des moyens (20, 21) pour perfuser au patient (9) un liquide de perfusion contenant une substance (A),

  caractérisé en ce qu'il comprend des moyens de dosage (22, 25) pour ajuster la concentration de la substance (A) dans le sang du patient (9) à une concentration souhaitée $[A]_{DES}$, compte tenu du transfert diffusif et/ou convectif de la substance (A) au travers de la membrane (4) de l'échangeur (1), ces moyens de dosage comprenant des moyens (22, 25) pour régler le débit du liquide de perfusion $(Q_A)$ en fonction du débit $(Q_{OUT})$ de liquide usé.

2. Rein artificiel selon la revendication 1, caractérisé en ce que le débit $(Q_A)$ du liquide de perfusion et le débit $(Q_{OUT})$ du liquide usé sont liés par la formule :

$$Q_A = \frac{[A]_{DES}}{[A]_{SOL}} \times Q_{OUT}$$

où $[A]_{SOL}$ est la concentration de la substance (A) dans le liquide de perfusion.

3. Rein artificiel selon la revendication 1, caractérisé en ce que le débit $(Q_A)$ du liquide de perfusion et le débit $(Q_{OUT})$ du liquide usé sont liés par la formule :

$$Q_A = \frac{[A]_{DES}}{[A]_{SOL}} \times Cl$$

où $[A]_{SOL}$ est la concentration de la substance (A) dans le liquide de perfusion et Cl est la clairance du rein artificiel pour la substance (A).

4. Rein artificiel selon une des revendications 1 à 3, caractérisé en ce que les moyens de perfusion comprennent une source de liquide (20) reliée par une canalisation (21) au circuit (5, 7) pour circulation extracorporelle de sang.

5. Rein artificiel selon une des revendications 1 à 4, caractérisé en ce que les moyens pour régler le débit du liquide de perfusion comprennent un organe de réglage de débit (22) et une unité de commande (25) pour piloter l'organe de réglage de débit (22).

6. Rein artificiel selon les revendications 4 et 5, caractérisé en ce que l'organe de réglage de débit est une pompe (22) disposée sur la canalisation (21).

7. Rein artificiel selon une des revendications 1 à 6, caractérisé en ce que la substance (A) est du bicarbonate.

8. Rein artificiel selon la revendication 7, caractérisé en ce qu'il comprend une source (10) de liquide de substitution ne contenant pas de bicarbonate reliée au circuit (5, 7) pour circulation extracorporelle de sang.

9. Rein artificiel selon une des revendications 7 et 8, caractérisé en ce qu'il comprend une source (10) de liquide de dialyse ne contenant pas de bicarbonate reliée à une entrée du second compartiment (3) de l'échangeur (1).

10. Rein artificiel selon la revendication 7, caractérisé en ce qu'il comprend une source (10) de liquide de dialyse/substitution ne contenant pas de bicarbonate reliée au circuit (5, 7) pour circulation extracorporelle de sang et à une entrée du second compartiment (3) de l'échangeur (1), et des moyens d'occlusion (15, 16 ; 29) pour ou bien isoler la source (10), ou bien permettre l'écoulement du liquide de dialyse/substitution dans le circuit (5, 7) pour circulation extracorporelle de sang, ou bien permettre l'écoulement du liquide de dialyse/substitution dans le second compartiment (3) de l'échangeur (1).

11. Rein artificiel selon une des revendications 1 à 10, caractérisé en ce qu'il comprend des moyens (19, 26) pour faire varier le débit d'ultrafiltration dans l'échangeur (1).

12. Rein artificiel selon une des revendications 1 à 11, caractérisé en ce qu'il comprend des moyens (23, 24, 25 ; 24, 27, 28, 25) pour faire un bilan du (ou des) liquide(s) introduit(s) dans le circuit (5, 7) pour circulation extracorporelle de sang et du liquide usé (ultrafiltrat et/ou liquide de dialyse usé) s'écoulant du second compartiment (3) de l'échangeur (1).

13. Rein artificiel selon la revendication 12, caractérisé en ce que les moyens pour faire le bilan des liquides comprennent une balance (23) pour peser un réservoir (10) constituant une source de liquide de dialyse/substitution et un réservoir (17) pour le recueil du liquide usé, et une balance (24) pour peser un réservoir (20) constituant une source de liquide contenant la substance (A).

14. Rein artificiel selon une les revendications 11 et 12, caractérisé en ce qu'il comprend des moyens (25) pour commander les moyens (19, 26) pour faire varier le débit d'ultrafiltration en fonction du bilan des liquides et d'une consigne de débit $(Q_{WL})$ de perte de poids donne.

**Claims**

1. An artificial kidney comprising:

- an exchanger (1) having two compartments (2, 3) separated by a semipermeable membrane (4), a first compartment (2) being connected to a circuit (5, 7) for conveying a flow of blood outside the body, a second compartment (3) having an outlet for draining a waste liquid;

- means (20, 21) for perfusing to the patient (9) a perfusion liquid containing a substance (A), characterized in that it comprises dosage means (22, 25) for adjusting the concentration of the substance (A) in the blood of the patient (9) to a desired concentration $[A]_{DES}$, whereby the diffusive and/or convective transfer of the substance (A) through the membrane (4) of the exchanger (1) is taken into account, the dosage means comprising means (22, 25) for adjusting the flow rate ($Q_A$) of the perfusion solution as a function of the flow rate ($Q_{OUT}$) of the waste liquid.

2. An artificial kidney according to claim 1, characterized in that the flow rate ($Q_A$) of the perfusion liquid and the flow rate ($Q_{OUT}$) of the waste liquid are related by the equation :

$$Q_A = \frac{[A]_{DES}}{[A]_{SOL}} \times Q_{OUT}$$

where $[A]_{SOL}$ is the concentration of the substance (A) in the perfusion liquid.

3. An artificial kidney according to claim 1, characterized in that the flow rate ($Q_A$) of the perfusion liquid and the flow rate ($Q_{OUT}$) of the waste liquid are related by the equation :

$$Q_A = \frac{[A]_{DES}}{[A]_{SOL}} \times Cl$$

where $[A]_{SOL}$ is the concentration of the substance (A) in the perfusion liquid and Cl is the clearance of the artificial kidney for the substance (A).

4. An artificial kidney according to one of the claims 1 to 3, characterized in that the perfusion means comprises a source of liquid (20) connected by a duct (21) to the circuit (5, 7) for circulation of blood outside the body.

5. An artificial kidney according to one of the claims 1 to 4, characterized in that the means for adjusting the flow rate of the perfusion liquid comprises a flow rate adjusting member (22) and a control unit (25) for controlling the flow rate adjusting member (22).

6. An artificial kidney according to one of the claims 1 and 5, characterized in that the flow rate adjusting member is a pump (22) located on the duct (21).

7. An artificial kidney according to one of the claims 1 to 6, characterized in that the substance (A) is bicarbonate.

8. An artificial kidney according to claim 7, characterized in that it includes a source (10) of substitution liquid that does not contain bicarbonate connected to the circuit (5, 7) for conveying a flow of blood outside the body.

9. An artificial kidney according to one of the claims 7 and 8, characterized in that it includes a source (10) of dialysis liquid that does not contain bicarbonate connected to an inlet of the second compartment (3) of the exchanger (1).

10. An artificial kidney according to claim 7, characterized in that it includes a source (10) of substitution/dialysis liquid that does not contain bicarbonate connected to the circuit (5, 7) for conveying a flow of blood outside the body and to an inlet of the second compartment (3) of the exchanger (1), and blocking means (15, 16 ; 29) for either isolating the source (10), or enabling the substitution/dialysis liquid to flow out into the circuit (5, 7) for conveying a flow of blood outside the body, or else for allowing the substitution/dialysis liquid to flow into the second compartment (3) of the exchanger (1).

11. An artificial kidney according to one of the claims 1 to 10, characterized in that it includes means (19, 26) for varying the ultrafiltration flow rate through the exchanger (1).

12. An artificial kidney according to one of the claims 1 to 11, characterized in that it includes means (23, 24, 25 ; 24, 27, 28, 25) for measuring the difference between the amount of liquid(s) injected into the circuit (5, 7) for conveying a flow of blood outside the body and the amount of waste liquid (ultrafiltrate and/or waste dialysis liquid) flowing out from the second compartment (3) of the exchanger (1).

13. An artificial kidney according to claim 12, characterized in that the means for measuring the difference between the amounts of liquids comprises scales (23) for weighing a container (10) constituting a source of substitution/dialysis liquid and a container (17) for collecting waste liquid, and scales (24) for weighing a container (20) constituting a source of solution containing the substance (A).

14. An artificial kidney according to one of the claims 11 and 12, characterized in that it includes means (25) for controlling the means (19, 26) for varying the ultrafiltration flow rate as a function of the difference between the liquids and a given reference value for weight loss flow rate ($Q_{WL}$).

**Patentansprüche**

1. Künstliche Niere, die folgendes umfaßt :

- einen Austauscher (1) mit zwei Kammern (2, 3), die durch eine halbdurchlässige Membran (4) getrennt sind, wobei eine erste Kammer (2) an einen Kreislauf (5, 7) für die extrakorporale Blutzirkulation angeschlossen ist, der mit einem Patienten (9) verbunden werden kann, und wobei eine zweite Kammer (3) über einen Ausgang zum Abführen einer verbrauchten Flüssigkeit verfügt;

- Vorrichtungen (20, 21), um dem Patienten (9) in Form einer Perfusion eine Perfusionsflüssigkeit zuzuführen, die eine Substanz (A) enthält, dadurch gekennzeichnet, daß sie Dosiervorrichtungen (22, 25) umfaßt, um die Konzentration der Substanz (A) im Blut des Patienten (9) auf eine gewünschte Konzentration $[A]_{DES}$ einzustellen, unter Berücksichtigung des diffusiv und / oder konvektiv erfolgenden Durchtritts der Substanz (A) durch die Membran (4) des Austauschers (1), wobei diese Dosiervorrichtungen jeweils Mittel (22, 25) umfassen, um eine Regulierung der Durchflußmenge der Perfusionsflüssigkeit $(Q_A)$ entsprechend der Durchflußmenge $(Q_{OUT})$ der verbrauchten Flüssigkeit vorzunehmen.

2. Künstliche Niere gemäß Anspruch 1, dadurch gekennzeichnet, daß die Durchflußmenge $(Q_A)$ der Perfusionsflüssigkeit und die Durchflußmenge $(Q_{OUT})$ der verbrauchten Flüssigkeit miteinander durch folgende Formel verknüpft sind :

$$Q_A = \frac{[A]_{DES}}{[A]_{SOL}} \times Q_{OUT}$$

Hierbei ist $[A]_{SOL}$ die Konzentration der Substanz (A) in der Perfusionsflüssigkeit.

3. Künstliche Niere gemäß Anspruch 1, dadurch gekennzeichnet, daß die Durchflußmenge $(Q_A)$ der Perfusionsflüssigkeit und die Durchflußmenge $(Q_{OUT})$ der verbrauchten Flüssigkeit miteinander durch folgende Formel verknüpft sind :

$$Q_A = \frac{[A]_{DES}}{[A]_{SOL}} \times Q_{OUT}$$

Hierbei ist $[A]_{SOL}$ die Konzentration der Substanz (A) in der Perfusionsflüssigkeit, und Cl ist die Clearance der künstlichen Niere für die Substanz (A).

4. Künstliche Niere gemäß einem der Ansprüche 1 bis 3, dadurch gekennzeichnet, daß die Perfusionsvorrichtungen eine Flüssigkeitsquelle (20) umfassen, die über eine Leitung (21) an den Kreislauf (5, 7) für die extrakorporale Blutzirkulation angeschlossen ist.

5. Künstliche Niere gemäß einem der Ansprüche 1 bis 4, dadurch gekennzeichnet, daß die Vorrichtungen zum Regulieren der Durchflußmenge der Perfusionsflüssigkeit ein Element zum Regulieren der Durchflußmenge (22) und eine Steuereinheit (25) zum Steuern des Elements zum Regulieren der Durchflußmenge (22) umfassen.

6. Künstliche Niere gemäß den Ansprüchen 4 und 5, dadurch gekennzeichnet, daß das Element zum Regulieren der Durchflußmenge eine Pumpe (22) ist, die sich an der Leitung (21) befindet.

7. Künstliche Niere gemäß einem der Ansprüche 1 bis 6, dadurch gekennzeichnet, daß es sich bei der Substanz (A) um Bikarbonat handelt.

8. Künstliche Niere gemäß Anspruch 7, dadurch gekennzeichnet, daß sie eine Quelle (10) für eine Substitutionsflüssigkeit umfaßt, die kein Bikarbonat enthält, wobei diese Quelle an den Kreislauf (5, 7) für die extrakorporale Blutzirkulation angeschlossen ist.

9. Künstliche Niere gemäß einem der Ansprüche 7 und 8, dadurch gekennzeichnet, daß sie eine Quelle (10) für eine Dialyseflüssigkeit umfaßt, die kein Bikarbonat enthält, wobei diese Quelle an einen Eingang der zweiten Kammer (3) des Austauschers (1) angeschlossen ist.

10. Künstliche Niere gemäß Anspruch 7, dadurch gekennzeichnet, daß sie eine Quelle (10) für die Dialyse- / Substitutionsflüssigkeit umfaßt, die kein Bikarbonat enthält und an den Kreislauf (5, 7) für die extrakorporale Blutzirkulation und an einen Eingang der zweiten Kammer (3) des Austauschers (1) angeschlossen ist, und daß sie Absperrvorrichtungen (15, 16; 29) umfaßt, um entweder die Quelle (10) abzusperren oder aber das Ablaufen der Dialyse- / Substitutionsflüssigkeit in den Kreislauf (5, 7) für die extrakorporale Blutzirkulation zu ermöglichen, oder aber um das Ablaufen der Dialyse- / Substitutionsflüssigkeit in die zweite Kammer (3) des Austauschers (1) zu ermöglichen.

11. Künstliche Niere gemäß einem der Ansprüche 1 bis 10, dadurch gekennzeichnet, daß sie Vorrichtungen (19, 26) umfaßt, um die Ultrafiltrations-Durchflußmenge im Austauscher (1) zu verändern.

12. Künstliche Niere gemäß einem der Ansprüche 1 bis 11, dadurch gekennzeichnet, daß sie Vorrichtungen (23, 24, 25; 24, 27, 28, 25) umfaßt, um eine Bilanz der Flüssigkeit / der Flüssigkeiten, die in den Kreislauf (5, 7) für die extrakorporale Blutzirkulation eingegeben wird / werden, sowie der verbrauchten Flüssigkeit (Ultrafiltrat und / oder verbrauchte Dialyseflüssigkeit), die aus der zweiten Kammer (3) des Austauschers (1) abläuft, zu erstellen.

13. Künstliche Niere gemäß Anspruch 12, dadurch gekennzeichnet, daß die Vorrichtungen, um die Bilanz der Flüssigkeiten zu erstellen, eine Waage (23) umfassen, um einen Behälter (10) zu wiegen, der eine Quelle für die Dialyse- / Substitutionsflüssigkeit darstellt, sowie einen Behälter (17) für das Auffangen der verbrauchten Flüssigkeit, und eine Waage (24) zum Wiegen eines Behälters (20), der eine Quelle jener Flüssigkeit darstellt, die die Substanz (A) enthält.

14. Künstliche Niere gemäß einem der Ansprüche 11 und 12, dadurch gekennzeichnet, daß sie Vorrichtungen (25) zur Steuerung der Mittel (19, 26) umfaßt, um die Ultrafiltrations-Durchflußmenge entsprechend der Bilanz der Flüssigkeiten und einem Vorgabewert für eine bestimmte Durchflußmenge ($Q_{WL}$) des Gewichtsverlusts zu verändern.

Fig. 1

Fig. 2